# EUROPÄISCHE PATENTSCHRIFT

(11) **EP 0 752 470 B1**
(45) Veröffentlichungstag und Bekanntmachung des Hinweises auf die Patenterteilung: **25.09.2002**
(21) Anmeldenummer: 96110354.6
(22) Anmeldetag: 27.06.1996
(51) Int. Cl.: C12M 1/06, C12N 1/10, C12M 3/02

(54) **Verfahren zur Massenkultivierung von Ciliaten**
Method for the mass growth of ciliates
Procédé de développement en masse de ciliates

(30) Priorität: 07.07.1995 DE 19524307
(43) Veröffentlichungstag der Anmeldung: 08.01.1997
(73) Patentinhaber: Celanese Ventures GmbH, 65926 Frankfurt am Main (DE)
(72) Erfinder: Kiy, Thomas, Dr., 65929 Frankfurt (DE); Marquardt, Rüdiger, Dr., 60389 Frankfurt (DE)
(74) Vertreter: Luderschmidt, Schüler & Partner GbR

(56) Entgegenhaltungen:
- US-A- 4 649 118
- US-A- 5 008 197
- PATENT ABSTRACTS OF JAPAN vol. 1996, no. 09, 30. September 1996 (1996-09-30) & JP 08 133980 A (HARIMA CHEM INC), 28. Mai 1996 (1996-05-28)
- PATENT ABSTRACTS OF JAPAN vol. 005, no. 164 (C-076), 21. Oktober 1981 (1981-10-21) & JP 56 095394 A (HIROUCHI SOGO), 1. August 1981 (1981-08-01)
- PATENT ABSTRACTS OF JAPAN vol. 006, no. 154 (C-119), 14. August 1982 (1982-08-14) & JP 57 074082 A (HITACHI PLANT ENG & CONSTR CO LTD), 10. Mai 1982 (1982-05-10)

## Beschreibung

Die Erfindung betrifft ein Verfahren zur Kultivierung von Ciliaten in Spinnerflaschen.

Ciliaten, stellen eine vielversprechende Quelle für biogene Wertstoffe, wie etwa Enzyme (I. Munro, 1985, Process Biochem., 20, 139), ungesättigte Fettsäuren (Y. Gosselin et al., 1989, Biotechnol. Lett., 11, 423) und "single cell protein" (A. Ayerbe, 1980, Enzyme Microb. Technol., 2, 54), dar.

Ein Grund dafür, daß Ciliaten trotz ihres Potentials bis heute als Produzenten nicht industriell genutzt werden, liegt in der häufig schlechten Züchtbarkeit dieser Organismen. Zu den zentralen Problemen bei der Fermentation von Ciliaten zählen der Mangel an geeigneten, preiswerten Kulturmedien und die häufig hohe Sensitivität gegenüber Scherkräften (Y. Gosselin et al., 1989, Biotechnol. Lett., 11, 423; M. Midler & R.K. Finn, 1966, Biotechnol. Bioeng., 8, 71). Während einige Ciliaten wie z.B. Tetrahymena und Paramecium erfolgreich in Fermentern kultiviert werden können (T. Kiy & A. Tiedtke, 1992, Appl. Microbiol. Biotechnol., 38, 141; U. Schönefeld et al., J. Protozool., 33, 222), existiert für die Mehrzahl der Ciliaten bislang keine Methode zur Massenkultivierung. Aber selbst die o.g. Gattungen lassen sich nicht unbeschadet in herkömmlichen Fermentern kultivieren. Dies läßt sich u.a. daran erkennen, daß der Grad der Schädigung von Tetrahymena-Zellen als Maß für die Scherkräfte in Rührfermentern herangezogen wurde (M. Midler & R.K. Finn, 1966, Biotechnol. Bioeng., 8, 71). Viele Ciliaten wurden bisher nur im sehr kleinen Maßstab, bei sehr geringen Zelldichten vermehrt. So ist die gängige Methode zur Zucht colpodider Bodenciliaten immer noch die "flooded petri dish"-Methode, wobei wenige ml Kultur in Petrischalen inkubiert werden (W. Foissner, 1993, Protozoenfauna Vol. 4/1: Colpodia (Cilipohora), Gustav Fischer Verl.).

Wünschenswert wäre eine Methode zur Kultivierung von Ciliaten in Massenkulturen. Solch ein System ist als Voraussetzung zur Beurteilung bzw. Nutzung dieser Organismen als Quelle biogener Wertstoffe zu sehen.

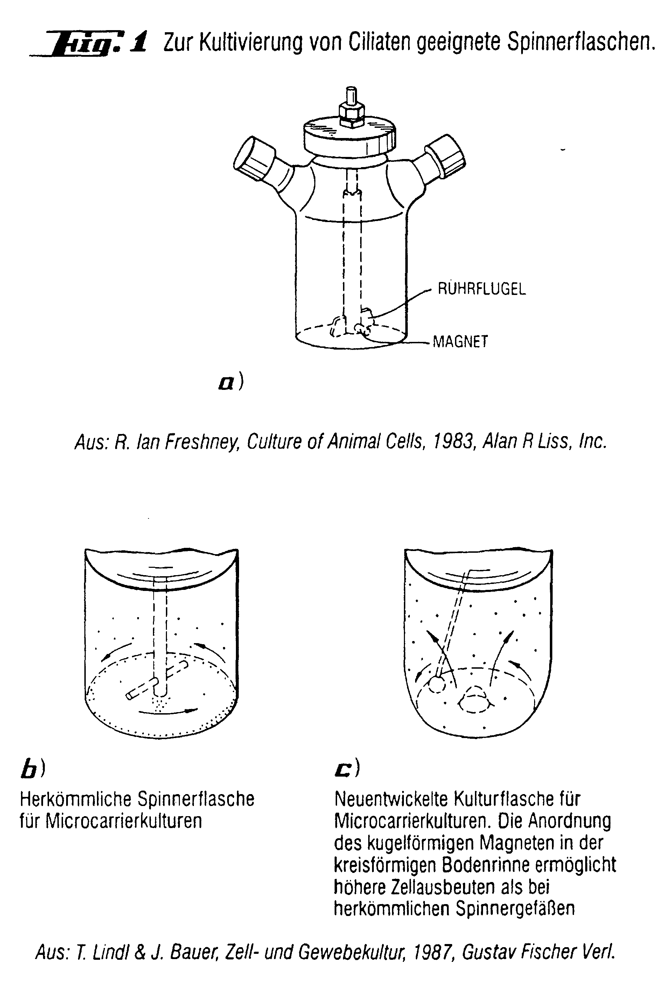

Die in herkömmlichen Fermentern auftretenden Scherkräfte werden von vielen Ciliaten nicht toleriert. Ein System, das relativ geringe Scherkräfte erzeugt, aber dennoch ausreichende Durchmischung der Kultur gewährleistet, ist die sogenannte Spinnerflasche (Fig. 1). Kennzeichnend für dieses System ist der Rührer mit Magnetkern, angetrieben durch einen unter dem Kulturgefäß befindlichen Magnetrührer. Der Rührer bewirkt eine schonende und scherstreßarme Durchmischung.

Obwohl diese Kultivierungsmethode bereits vor vielen Jahren zur Kultivierung von tierischen Zellkulturen (z.B. Hybridomazellen) entwickelt wurde (T. Lidl & J. Bauer, 1987, Zell- und Gewebekultur, Gustav-Fischer Verl.), ist es unterblieben, diese Kultivierungsmethode auf freilebende Ciliaten zu übertragen. Lediglich entfernt verwandte Eukaryoten wie Euglena aus der Gruppe der Euglenophyceen, Chlamydomonas aus der Gruppe der Chlorophyceen (Vogels et al., 1978, J. Phycol., 14, 403) und Entamoeba histolytica aus der Gruppe der Amoebida (Said-Fernandez, et al., 1992, Arch. Med. Res., 23, 57) wurden bereits in Spinnerflaschen kultiviert.

Überraschenderweise stellte sich heraus, daß sich diese Art der Zellkultivierung auf Ciliaten übertragen läßt.

Die vorliegende Erfindung betrifft somit ein Verfahren zur Kultivierung von Ciliaten unter Rühren des Kulturmediums, das sich dadurch auszeichnet, daß als Zuchtgefäß eine Kulturflasche, welche über einen mit einem Magnetkern versehenen Rührer verfügt, der im Kopfteil der Kulturflasche so aufgehängt ist, daß das Rührwerk den Flaschenboden nicht berührt, wobei der Rührer mittels eines Magnetfeldes angetrieben wird, vorzugsweise eine sogenannte Spinnerflasche, verwendet wird.

Alle bisher getesteten Ciliatenspezies konnten erfolgreich in solchen Spinnerflaschen gezüchtet werden. Insbesondere Vertreter der Colpodea (z.B. Colpoda, Platyophrya), der Holotrichia (z.B. Tetrahymena, Paramecium, Colpidium), der Peritrichia (z.B. Vorticella) und der Spirotrichia (z.B. Blepharisma, Stentor, Euplotes, Stylonichia), lassen sich hervorragend in Spinnerflaschen fermentieren. (Systematik nach K. Hausmann, 1985, Protozoologie, Georg Thieme Verl.).

Die Zuchttemperatur liegt dabei zwischen 13 und 35°C, vorzugsweise aber zwischen 20 und 30°C.

Die Ciliaten lassen sich in axenischen Medien (Tetrahymena z.B. auf PPYS-Medium bzw. Magermilchmedium), in monoxenischen Medien (Blepharisma z.B. mit Bacillus subtilis oder Pseudomonas fluorescens als Futterbakterium) oder in polyxenischen Medien züchten.

Die Kultivierung kann als Batch-Fermentation, Fed-Batch-Fermentation (regelmäßiges Zufüttern eines Substrat) oder Fermentation mit zyklischem Mediumaustausch durchgeführt werden.

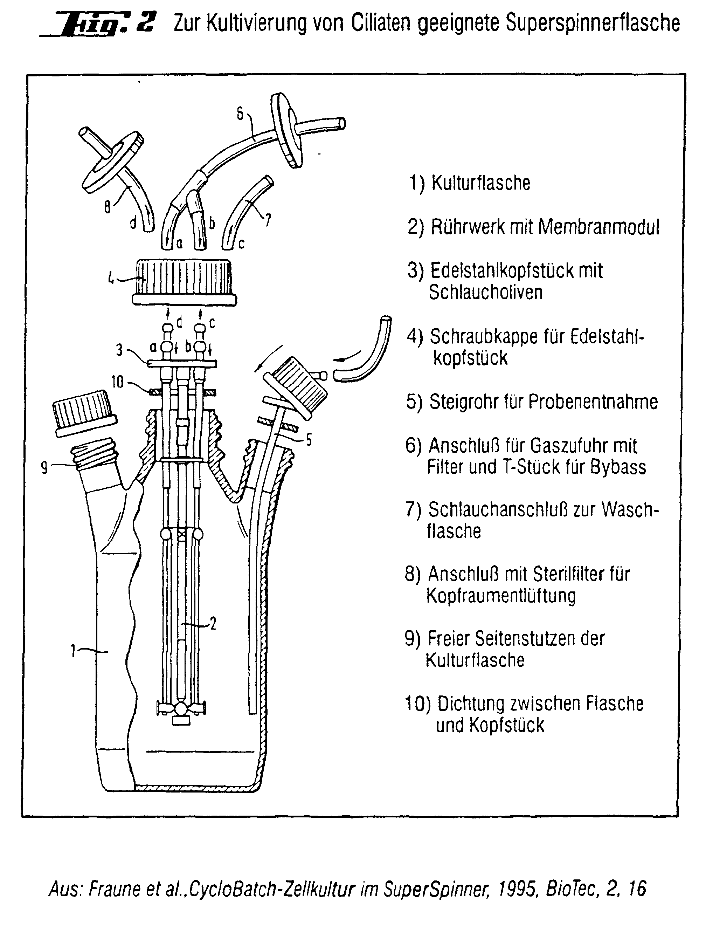

Neben den Spinnerflaschen (Fig. 1) eignen sich auch Superspinnerflaschen (Fig. 2), die durch einen Membranbegasungsrührer zur blasenfreien Begasung des Mediums gekennzeichnet sind, zur Kultivierung von Ciliaten, Superspinnerflaschen werden bisher zur Produktion monoklonaler Antikörper aus Hybridomazellen eingesetzt.

Der Rührmodus kann sowohl als Pendelmischtechnik als auch als Pendelmischtechnik mit Drehrichtungswechsel ausgelegt sein. Die Rührergeschwindigkeit liegt zwischen 1 und 70 upm, bevorzugt im Bereich 10 - 40 upm (upm = Umdrehungen pro Minute).

### Beispiele:

### (Sämtliche Spezies sind entweder über die American Culture Collection, Rockville, Maryland, USA oder die Culture Collection of Algae and protozoa, Ambleside, Cumbria, United Kingdom zugänglich).

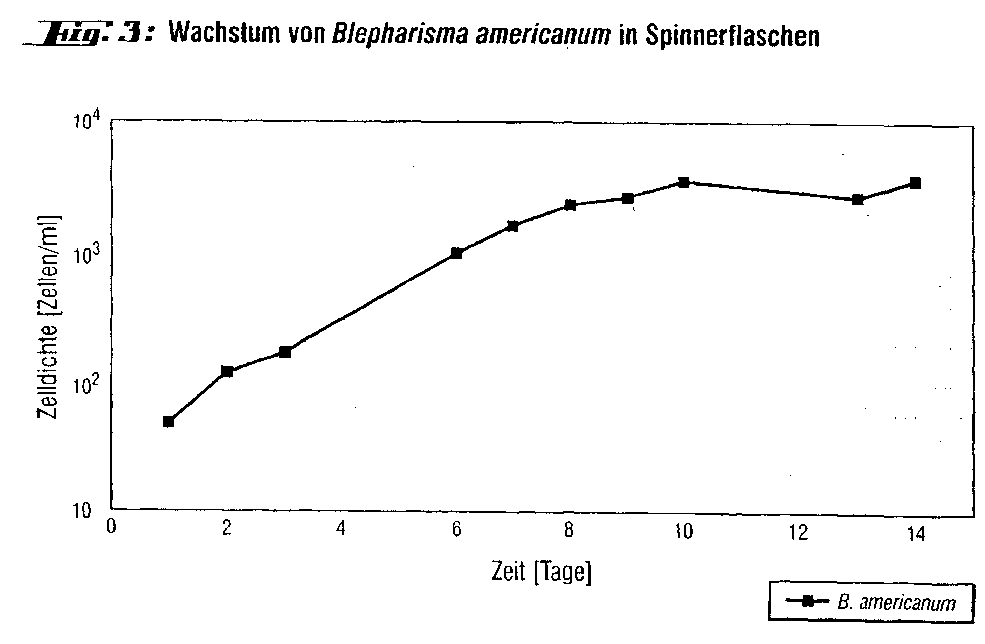

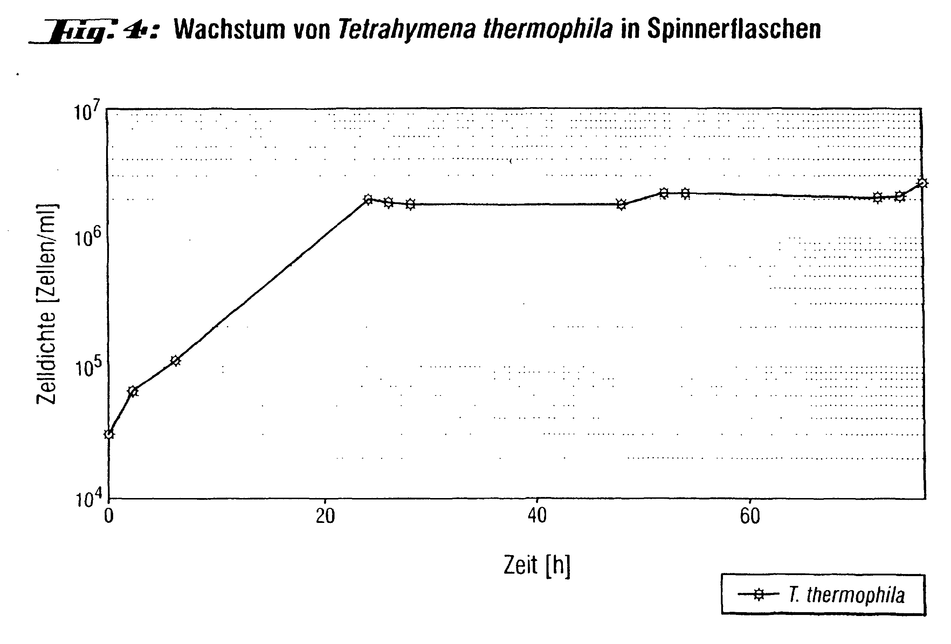

1. Blepharisma americanum wurde in einem 2-I-TECNOMARA® Cellspingefäß in 1 l Standardmedium (9 Teile Eau Volvic, 1 Teil Salatmedium, einige autoklavierte Weizenkörner) bei 25°C inkubiert. Die Rührgeschwindigkeit betrug 30 upm. Nach 14 Tagen hatten die Zellen eine Dichte von 3.600 Zellen/ml erreicht (Fig. 3). Durch wiederholtes Zufüttern (1x pro Woche) einer Bakteriensuspension (10 ml/500 ml; Stamm 12658 aus American Type Culture Collection) wurden Zelldichten von 50.000 Zellen/ml erreicht.
2. Colpoda cucullus wurde in einem 1-I-TECNOMARA® Cellspingefäß in 500 ml Standardmedium (9 Teile Eau Volvic, 1 Teil Salatmedium, einige autoklavierte Weizenkörner) bei 25°C inkubiert. Zusätzlich wurden 10 ml einer autoklavierten Bakteriensuspension (Stamm 12658 aus American Type Culture Collection) zugefüttert. Die Rührgeschwindigkeit betrug 30 upm, die Animpfdichte 100 Zellen/ml. Zelldichten von 10.000 Zellen/ml wurden nach 280 h ermittelt.
3. Colpoda steinii wurde in einem 1-I-TECNOMARA® Cellspingefäß in 500 ml S/W-Medium (CCAP-Katalog, 1988, titus Wilson & Son Ltd., Kendal) bei 25°C inkubiert. Die Rührgeschwindigkeit betrug 30 upm, die Animpfdichte 50.000 Zellen/ml. Zelldichten von 10⁷ Zellen/ml wurden nach 48 Stunden ermittelt.
4. Platyophrya macrostoma wurde in einem 2-I-TECNOMARA® Cellspingefäß in 700 ml Medium (9 Teile Eau Volvic, 1 Teil Salatmedium) unter Zusatz von 10 ml autoklavierter 10 %iger Hefesuspension bei 25°C inkubiert. die Rührgeschwindigkeit betrug 30 upm. Die Zellen erreichten nach 150 h eine Konzentration von 70.000 Zellen/ml. Die Animpfdichte betrug 10⁴ Zellen/ml.
5. Tetrahymena thermophila wurde in einem 1-I-TECNOMARA® Cellspingefäß in 500 ml Magemilchmedium (2 % Magermilchpulver, 0,5 % Hefeextrakt, 0,1 % Glucose, 0,003 % Sequestrene) bei 25°C kultiviert. Die Durchmischung erfolgte über Pendelmischtechnik mit Drehrichtungswechsel (30 upm). Nach 24 h hatten die Zellen eine Zelldichte von 2 x 10⁶ Zellen/ml erreicht (Fig. 4). Die Animpfdichte betrug 3 x 10⁴ Zellen/ml.
6. Tetrahymena setosa wurde in einem 1-I-TECNOMARA® Cellspingefäß in 500 ml Magermilchmedium (2 % Pharmamedia, 0,5 % Hefeextrakt, 0,1 % Glucose, 0,003 % Sequestrene) bei 25°C kultiviert. Die Durchmischung erfolgte über Pendelmischtechnik mit Drehrichtungswechsel (30 upm). Nach 24 h hatten die Zellen eine Zelldichte von 3 x 10⁶ Zellen/ml erreicht. Die Animpfdichte betrug 3 x 10⁴ Zellen/ml.
7. Colpidium campylum wurde in einem 1-I-TECNOMARA® Cellspingefäß in 500 ml Magermilchmedium (2 % Magermilchpulver, 0,5 % Hefeextrakt, 0,1 % Glucose, 0,003 % Sequestrene) bei 25°C kultiviert. Die Durchmischung erfolgte über Pendelmischtechnik mit Drehrichtungswechsel (30 rpm). Nach 72 h hatten die Zellen eine Zelldichte von 1,2 x 10⁶ Zellen/ml erreicht. Die Animpfdichte betrug 2,2 x 10⁴ Zellen/ml.

## Patentansprüche

1. Verfahren zur Kultivierung von Ciliaten unter Rühren des Kulturmediums, **dadurch gekennzeichnet, daß** als Zuchtgefäß eine Kulturflasche verwendet wird, welche über einen mit einem Magnetkern versehenen Rührer verfügt, der im Kopfteil der Kulturflasche so aufgehängt ist, daß das Rührwerk den Flaschenboden nicht berührt, wobei der Rührer mittels eines Magnetfeldes angetrieben wird.

2. Verfahren nach Anspruch 1, **dadurch gekennzeichnet, daß** der Rührer als Membranbegasungsrührer ausgestaltet ist.

3. Verfahren nach einem der Ansprüche 1 oder 2, **dadurch gekennzeichnet, daß** die Kultivierung als Fed-Batch-Fermentation durchgeführt wird.

4. Verfahren nach einem der Ansprüche 1 oder 2, **dadurch gekennzeichnet, daß** die Kultivierung unter zyklischem Mediumaustausch durchgeführt wird.

5. Verfahren nach einem der Ansprüche 1 bis 4, **dadurch gekennzeichnet, daß** die Rührgeschwindigkeit 1 - 70 upm beträgt.

6. Verfahren nach Anspruch 5, **dadurch gekennzeichnet, daß** die Rührgeschwindigkeit 10 - 40 upm beträgt.

7. Verfahren nach einem der Ansprüche 1 bis 6, **dadurch gekennzeichnet, daß** der Rührmodus als Pendelmischtechnik ausgelegt ist.

8. Verfahren nach Anspruch 7, **dadurch gekennzeichnet, daß** der Rührmodus als Pendelmischtechnik mit Drehrichtungswechsel ausgelegt ist.

9. Verfahren nach einem der Ansprüche 1 bis 8, **dadurch gekennzeichnet, daß** die Ciliaten der Gruppe der Colpodia angehören.

10. Verfahren nach einem der Ansprüche 1 bis 8, **dadurch gekennzeichnet, daß** die Ciliaten der Gruppe der Holotrichia angehören.

11. Verfahren nach einem der Ansprüche 1 bis 8, **dadurch gekennzeichnet, daß** die Ciliaten der Gruppe der Peritrichia angehören.

12. Verfahren nach einem der Ansprüche 1 bis 8, **dadurch gekennzeichnet, daß** die Ciliaten der Gruppe der Spirotrichia angehören.

## Claims

1. Procedure for the cultivation of ciliates by stirring the culture medium, **characterised in that** a culture bottle is used as the growth vessel which is provided with a stirrer having a magnetic core, which is so suspended from the head-piece of the culture bottle that the stirrer does not touch the bottom of the bottle, where the stirrer is driven by a magnetic field.

2. Procedure in accordance with Claim 1, **characterised in that** the stirrer is designed to function as a membrane gas dispersion stirrer.

3. Procedure in accordance with one of the Claims 1 or 2, **characterised in that** the cultivation is carried out as Fed-Batch-Fermentation.

4. Procedure in accordance with one of the Claims 1 or 2, **characterised in that** the cultivation is carried out under cyclic exchange of the medium.

5. Procedure in accordance with one of the Claims 1 to 4, **characterised in that** the stirring speed ranges between 1 and 70 rpm.

6. Procedure in accordance with Claim 5, **characterised in that** the stirring speed ranges between 10 and 40 rpm.

7. Procedure in accordance with one of the Claims 1 to 6, **characterised in that** the stirring mode provides a facility for oscillation mixing.

8. Procedure in accordance with Claim 7, **characterised in that** the stirring mode provides a facility for oscillation mixing with alternating directions of rotation.

9. Procedure in accordance with one of the Claims 1 to 8, **characterised in that** the ciliates belong to the group of Colpodia.

10. Procedure in accordance with one of the Claims 1 to 8, **characterised in that** the ciliates belong to the group of Holotrichia.

11. Procedure in accordance with one of the Claims 1 to 8, **characterised in that** the ciliates belong to the group of Peritrichia.

12. Procedure in accordance with one of the Claims 1 to 8, **characterised in that** the ciliates belong to the group of Spirotrichia.

## Revendications

1. Procédé de culture de ciliés sous agitation du milieu de culture, **caractérisé en ce que** l'on utilise, en tant que récipient de culture, une bouteille de culture, qui dispose d'un agitateur pourvu d'un noyau magnétique, lequel agitateur est suspendu dans la partie de tête de la bouteille de culture de telle sorte que l'agitateur ne touche pas le fond de la bouteille, l'agitateur étant entraîné à l'aide d'un champ magnétique.

2. Procédé selon la revendication 1, **caractérisé en ce que** l'agitateur est réalisé sous la forme d'un agitateur à gazage par membrane.

3. Procédé selon l'une des revendications 1 ou 2, **caractérisé en ce que** la culture est effectuée en tant que fermentation "fed-batch".

4. Procédé selon l'une des revendications 1 ou 2, **caractérisé en ce que** la culture est effectuée dans le cadre d'un échange cyclique des milieux.

5. Procédé selon l'une quelconque des revendications 1 à 4, **caractérisé en ce que** la vitesse. d'agitation est de 1 à 70 révolutions par minuté.

6. Procédé selon la revendication 5, **caractérisé en ce que** la vitesse d'agitation est de 10 à 40 rpm.

7. Procédé selon l'une quelconque des revendications 1 à 6, **caractérisé en ce que** le mode d'agitation est réalisé en tant que technique de mélange à oscillations.

8. Procédé selon la revendication 7, **caractérisé en ce que** le mode d'agitation est réalisé en tant que technique de mélange à oscillations avec changement du sens de rotation.

9. Procédé selon l'une quelconque des revendications 1 à 8, **caractérisé en ce que** les ciliés appartiennent au groupe des colpodia.

10. Procédé selon l'une quelconque des revendications 1 à 8, **caractérisé en ce que** les ciliés appartiennent au groupe des holotrichia.

11. Procédé selon l'une quelconque des revendications 1 à 8, **caractérisé en ce que** les ciliés appartiennent au groupe de péritrichia.

12. Procédé selon l'une quelconque des revendications 1 à 8, **caractérisé en ce que** les ciliés appartiennent au groupe des spirotrichia.
